# EUROPEAN PATENT APPLICATION

(11) **EP 2 208 501 A1**
(43) Date of publication of application: **21.07.2010**
(21) Application number: 09000675.0
(22) Date of filing: 19.01.2009
(51) Int. Cl.: A61K 47/48

(54) **Anticancer prodrug sensitive to target protease**

(30) Priority: 14.01.2009 KR 20090003108
(71) Applicant: University of Ulsan Foundation For Industry Cooperation, Ulsan 680-190 (KR)
(72) Inventor: Kim, Sang Yoon, Songpa-gu, seoul 138-220 (KR); Lee, Beom Suk, Jung-gu Seoul 100-781 (KR)
(74) Representative: Roos, Peter

(57) **Abstract**

Provided is an anticancer prodrug sensitive to a target protease. The anticancer prodrug includes an anticancer drug, peptide that is specifically decomposed by the target protease excessively secreted by cancer cells, and a polymer that is specifically accumulated at a target cancer site. When the inactive anticancer prodrug is administered, the anticancer prodrug is accumulated at the target caner site and then the peptide is decomposed by irradiation of radioactive rays, thereby releasing an active anticancer drug at the target caner site. Accordingly, destruction of normal cells can be minimized and a high anticancer therapeutic effect can be obtained by using a small amount of the anticancer drug administered and a small amount of radioactive rays irradiated.

Sequence Listing

<110>	UNIVERSITY OF ULSAN FOUNDATION FOR INDUSTRY COOPERATION

<120>	Anticancer prodrug sensitive to target protease

<130>	op-2009-0002

<160>	1

<170>	KopatentIn 1.71

<210>	1
<211>	8
<212>	PRT
<213>	protein specific to caspase protease

<400>	1
Cys Asp Glu Val Glu Ala Pro Lys
  1               5

## Description

### CROSS-REFERENCE TO RELATED PATENT APPLICATIONS

This application claims the benefit of Korean Patent Application No. 10-2009-0003108, filed on January 14, 2009, in the Korean Intellectual Property Office, the disclosure of which is incorporated herein in its entirety by reference.

### BACKGROUND OF THE INVENTION

### 1. Field of the Invention

The present invention relates to an anticancer prodrug sensitive to target protease that minimizes destruction of normal cells and increases an anticancer therapeutic effect by using a small amount of an anticancer drug administered and a small amount of radioactive rays irradiated.

### 2. Description of the Related Art

Currently, cancer is the most common cause of death from worldwide. In addition, environmental problems, longer life expectances, and westernized diets will lead to an increase in cancer patients in the future. Typically, cancer is treated by using a conventional anticancer drug together with radiotherapy.

Most of the chemoradiation therapies kill cancer cells mainly by inducing cancer cell apoptosis. In addition, when radiotherapy is used together with conventional anticancer drugs that are used in conventional chemotherapies, higher therapeutic effects can be obtained.

However, although conventional anticancer treatments and radiotherapies show high efficacy on cancer cell growth suppression and cancer cell apoptosis, normal cells as well as cancer cells are affected due to a large amount of anticancer drugs administered and a large amount of radioactive rays irradiated, thereby causing serious side effects.

In response to these problems, target therapies have been recently developed. Target therapies have different mechanisms from conventional chemotherapies and hormone therapies, and do not affect normal cells and only target the cell growth signal delivery, receptor, and genetic mutation of tumor cells. Therefore, target therapies show high drug efficacy and lowest levels of cytotoxicity can be obtained.

Meanwhile, even when an anticancer treatment is performed together a recently developed molecular imaging technique in which a destination tumor is precisely targeted, it is very important to optimize amounts of anticancer drug administered and radioactive rays used in radiotherapies to obtain high anticancer therapeutic effect.

All cancer cells can be destroyed by administering appropriate amounts of anticancer drug and irradiating appropriate amounts of radioactive rays. However, in this case, normal cells located near cancer cells can also be damaged. Accordingly, research into selective anticancer treatments and physical methods of accurately irradiating radioactive rays to a target site are actively being conducted.

Thus, if high cell apoptosis effects can be obtained by using a target anticancer drug that minimizes damages on normal cells and a low amount of radioactive rays, conventional chemotherapy and radiotherapy effects can be substantially increased.

### SUMMARY OF THE INVENTION

To solve problems of conventional techniques, the inventors of the present application used various innovation techniques together, such as a drug delivery technique, a nano medical technique, and radiotherapy, to develop a future-oriented anticancer prodrug sensitive to target protease that maximizes a therapeutic efficiency and minimize side effects, and completed the present invention.

Accordingly, the present invention provides an anticancer prodrug sensitive to target protease that minimizes destruction of normal cells and increases an anticancer therapeutic effect by using a small amount of an anticancer drug administered and a small amount of radioactive rays irradiated.

According to an aspect of the present invention, there is provided an anticancer prodrug sensitive to target protease. The anticancer prodrug sensitive to target protease includes an anticancer drug, a peptide and a polymer, which are bound to each other. In this case, the peptide is specifically decomposed by the target protease that is excessively secreted by cancer cells, and the polymer is accumulatable specifically at a cancer site.

The anticancer prodrug may further include a linker that bonds the peptide bound to the anticancer drug to the polymer, thereby forming an anticancer drug-peptide-linker-albumin composite.

The linker may be any linker that has hydroxysuccinimide or maleimide at its end. Examples of the linker include succinimidyl-4-(N-maleimidomethyl)cyclohexane-1-carboxylate, bis N-hydroxysuccinimide, and bis maleimide.

The anticancer prodrug is administered in an inactive state to a target site and accumulated, and then the peptide is decomposed by administration of a small amount of the anticancer drug and irradiation of a small amount of radioactive rays, thereby locally releasing the anticancer drug at the target site. In this case, the anticancer drug is an active component.

The target protease may be any one selected from the group consisting of caspases, matrix metalloproteinases (MMP), thrombin, FXIIIa, caspase, urokinase plasminogen activator (uPA), HIV protease, DPP-IV and proteasome.

The anticancer drug may be any one selected from the group consisting of doxorubicin, paclitaxel, adriamycin, cisplatin, 5-fluorouracil, mitomycin, chlomomycin, bleomycin, peplomycin, daunorubicin, aclarrubicin, neocarzinostatin, epirubicin, idarubicin and pirarubicin.

The peptide may be acetyloctapeptide including a peptide set forth in SEQ ID NO. 1. The target protease may be a caspase-3 protease.

The polymer may be any polymer having biocompatibility. For example, the polymer may be a biopolymer that is very effectively accumulated on cancer tissues and is used as a drug delivery carrier of an anticancer drug. Examples of the biopolymer include collagen, gelatin, dextran, poly-L-lysine, and poly-aspartic acid. The polymer may also be a synthesized polymer. Examples of the synthesized polymer include poly(N-2-(hydroxypropyl)methacrylamide, poly(divinyl ether-co-maleic anhydride), poly(styrene-co-maleic anhydride), and poly(ethylene glycol).

### BRIEF DESCRIPTION OF THE DRAWINGS

The above and other features and advantages of the present invention will become more apparent by describing in detail exemplary embodiments thereof with reference to the attached drawings in which:
FIG. 1 is a schematic view of an anticancer prodrug sensitive to target protease according to the present invention;
FIG. 2 is a schematic view to explain a mechanism of an anticancer prodrug sensitive to target protease according to the present invention;
FIG. 3 illustrates a synthesis example of an anticancer prodrug sensitive to target protease according to the present invention;
FIG. 4 illustrates inactive cytotoxicity of an anticancer prodrug sensitive to target protease according to the present invention; and

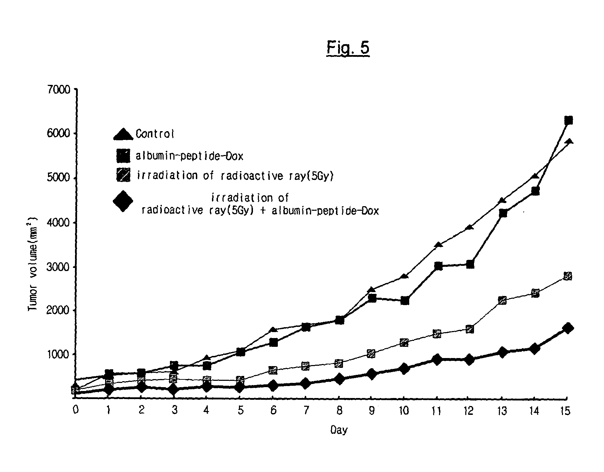

FIG. 5 illustrates an anticancer effect of an anticancer prodrug sensitive to target protease according to the present invention.

### DETAILED DESCRIPTION OF THE INVENTION

The present invention will now be described more fully with reference to the accompanying drawings, in which exemplary embodiments of the invention are shown.

In an anticancer prodrug according to the present invention, a polymer is chemically bound to an anticancer drug. Accordingly, the anticancer drug exists in an inactive state. In addition, since the anticancer prodrug is in a form of polymer nanoparticles, when injected into body, noninvasive and selective accumulation at an inflammatory site, such as a cancer tissue, can be achieved due to an enhanced permeability retention (EPR) effect. The EPR effect refers to an effect of increasing the amount of nanoparticles absorbed at a tumor site and a time period for which nanoparticles stay at the tumor site.

When the anticancer prodrug according to the present invention is administered and then, an anticancer drug in an amount with which side effects do not occur and a small therapeutic effect occur is administered and radioactive rays are irradiated, an insufficient cell apoptosis effect occurs at a cancer tissue site.

If the cell apoptosis effect occurs in a general condition, a distinctive anticancer therapeutic effect may not be obtained. However, if the cell apoptosis effect occurs after the anticancer prodrug according to the present invention is accumulated at a cancer tissue site, the inactive anticancer drug is activated. Accordingly, the activated anticancer drug is selectively amplified in phases at the cancer cell site and shows high levels of cytotoxicity and ultimately, a high target therapeutic efficiency.

Thus, this method can minimize damages on normal cells and increase an anticancer therapeutic effect by using a substantially small amount of anticancer drug administered and a small amount of radioactive rays irradiated, thereby significantly reducing side effects of radiotherapy performed together with anticancer drugs. In addition, only cancer cells are targeted and the anticancer drug is specifically activated during cell apoptosis to show additional cytotoxicity with respect to cancer cells. Accordingly, this method may contribute to an increase in a therapeutic effect of chemotherapy performed with a conventional radiotherapy.

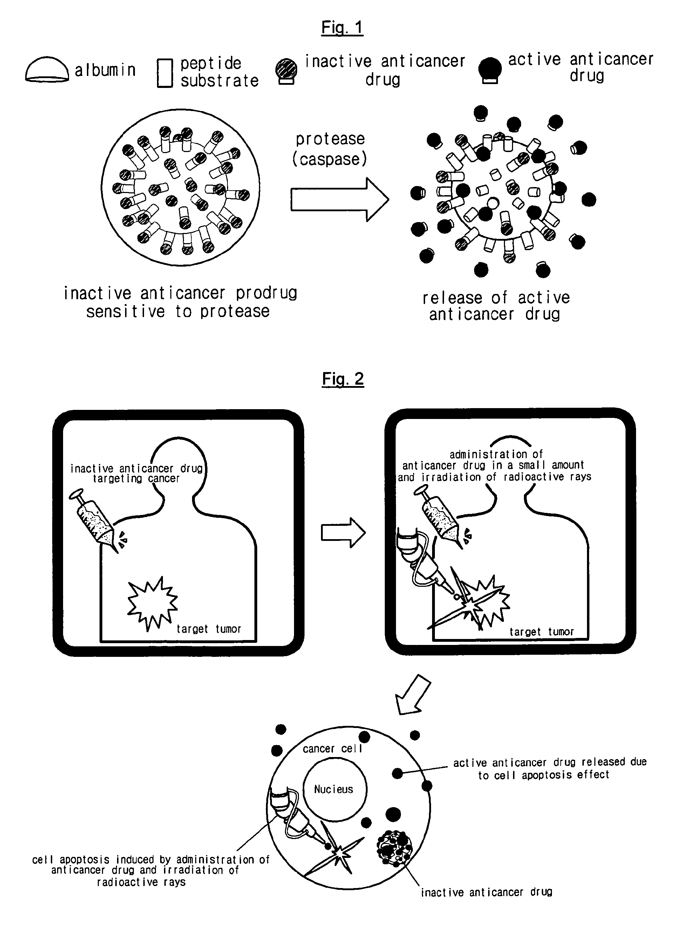

FIG. 1 is a schematic view of an anticancer prodrug sensitive to target protease according to the present invention, wherein the anticancer prodrug includes a protein, a peptide substrate and anticancer drug and is in a form of nanoparticles. Referring to FIG. 1, the anticancer drug is bound to the protein and exists in an inactive state. In this case, the protein may be albumin. However, when the peptide substrate is specifically decomposed by a protease secreted due to a cell apoptosis effect, an activated anticancer drug is released.

FIG. 2 is a schematic view to explain a mechanism of the anticancer prodrug sensitive to target protease according to the present invention. Referring to 1) in FIG. 2, when the anticancer prodrug according to the present invention is administered, the inactive anticancer drug is selectively accumulated at a target tumor. Then, as illustrated in 2) and 3) in FIG. 2, an anticancer drug that is less likely cause side effects and has low levels of cytotoxicity is administered and radioactive rays are irradiated. As a result, cancer cell apoptosis is induced and caspase may be excessively expressed due to the cell apoptosis. Therefore, the inactive anticancer drug bound to albumin is released by the expressed caspase and thus, the released and activated anticancer drug selectively destroy cancer cells.

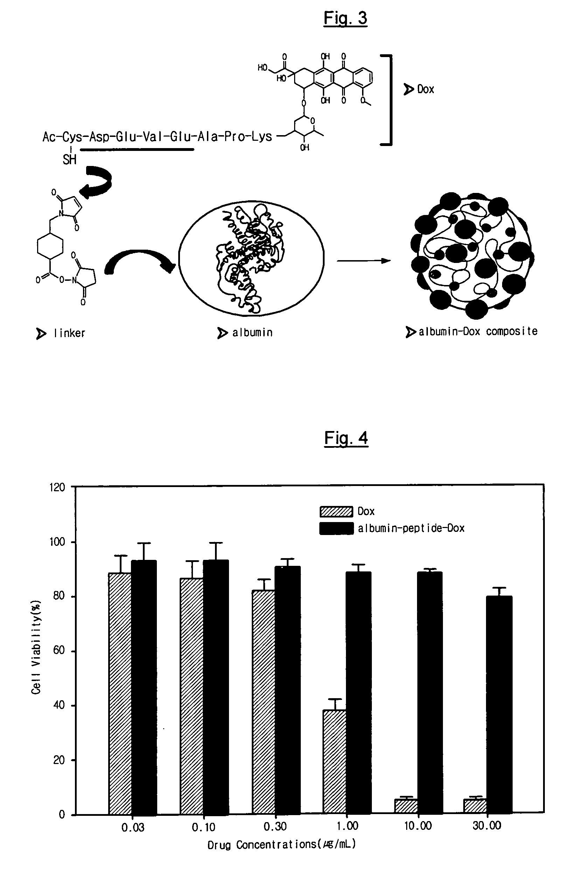

FIG. 3 illustrates a synthesis example of an anticancer prodrug sensitive to target protease according to the present invention. Referring to FIG. 3, Doxorubicin(Dox) as a model anticancer drug is chemically bound to acetyl octapeptide(Ac-Cys-Asp-Glu-Val-Glu-Ala-Pro-Lys) substrate including peptide (Cys-Asp-Glu-Val-Glu-Ala-Pro-Lys) that is set forth in SEQ ID NO.1 and specifically decomposed by caspase protease, thereby forming a peptide-Dox composite(Ac-Cys-Asp-Glu-Val-Glu-Ala-Pro-Lys-Dox).

The peptide-anticancer drug is chemically bound to albumin protein via a linker, thereby forming an albumin-peptide-anticancer drug that is an inactive anticancer prodrug. The albumin-peptide-anticancer drug is formed in a form of nanoparticles. The inactive anticancer prodrug, specifically, Asp-Glu-Val-Glu of the peptide substrate of the inactive anticancer prodrug is specifically decomposed by caspase-3 protease secreted during cell apoptosis and the activated Ala-Pro-Lys-Dox is released.

FIG. 4 illustrates inactive cytotoxicity of the albumin-peptide-Dox.

When the anticancer prodrug is manufactured as a pharmaceutical composition, the anticancer prodrug may further include a carrier, an excipient, or a diluent, which are conventionally used in a pharmaceutical composition.

Examples of available carrier, excipient, and diluent include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and various minerals.

The anticancer drug used in the present invention may be formed into various formulations according to respective conventional methods. For example, the anticancer drug may be formed in an oral formulation, an external-use formulation, a suppository formulation, or a sterilized injection solution formulation. The oral formation may be a powder formulation, a granule formulation, a tablet formulation, a capsule formulation, a suspension formulation, an emulsion formulation, a syrup formulation, or an aerosol formulation.

These formulations may be formed by using a conventionally available diluent or excipient, such as filler, an extender, a binder, a wetting agent, a disintegrant, or a surfactant. Examples of a solid formulation for oral administration include a tablet, a pill, a dusting powder, a granule and a capsule. These solid formulations are manufactured by using at least one excipient selected from starch, calcium carbonate, sucrose, lactose, gelatin, and the like.

In addition, a lubricant, in addition to the excipient, may also be used. The lubricant may be magnesium stearate or talc. Examples of a liquid formulation for oral administration include a suspension, a solution, an emulsion and a syrup. The liquid formulation may further include, in addition to water that is a commonly available simple diluent or liquid paraffin, various excipients, such as a wetting agent, a sweetener, an aromatic, or a preservative. Examples of a formulation for parenteral administration are a sterilized aqueous solution, a water-insoluble solution, a suspension, an emulsion, a lyophilized formulation and a suppository. The non-aqueous solution formulation and the suspension formulation may be propylene glycol, polyethylene glycol, a plant oil such as olive oil, or injectable ester such as ethyloleate. A base for the suppository formulation may be witepsol, macrogol, tween 61, cacao butter, laurin butter, or glycerogellatin.

When the anticancer prodrug according to the present invention is used as a pharmaceutical composition, the amount of the anticancer drug-peptide-albumin composite contained in the pharmaceutical composition may vary according to the age, gender, and weight of a patient, and the pharmaceutical composition may be administered at least one time for one day. The administration amount may vary according to an administration route, a development degree of disease, a gender, a weight, and an age. Accordingly, the administration amount may not be limited in any respect.

The pharmaceutical composition may be administered to mammals, such as rats, mice, livestock, or humans, via various routes. The pharmaceutical composition may be administered by using known methods. For example, the pharmaceutical composition may be administered via the mouth or rectum, or a vein, muscle, subcutaneous, intrauterine epidural or intracerebroventricular injection.

The pharmaceutical composition according to the present invention relates to a novel formulation of a conventional anticancer drug that has secured safety. Accordingly, the pharmaceutical composition according to the present invention is also stable.

The present invention will be described in further detail with reference to the following examples. These examples are for illustrative purposes only and are not intended to limit the scope of the present invention.

### <Synthesis Example 1> Preparation of anticancer prodrug sensitive to target protease

27 mg of bis (N-hydroxysuccinimide ester) (NHS), N-methylmorpholine, and 4-dimethylaminopyridin were reacted with 20 mg of doxorubicin (Dox) in the presence of dimethylformamide, thereby preparing a Dox including NHS (Dox-NHS).

10 mg of Dox-NHS prepared was reacted with 24 mg of acetyl octapeptide(Ac-Cys-Asp-Glu-Val-Glu-Ala-Pro-Lys) substrate including peptide that is set forth in SEQ ID No. 1 and specifically decomposed by caspase protease in dimethylformamide including N-methylmorpholine and dimethylaminopyridine for 6 hours, thereby forming a chemically bound peptide-Dox composite(Ac-Cys-Asp-Glu-Val-Glu-Ala-Pro-Lys-Dox).

Succinimidyl-4-(N-maleimidomethyl)cyclohexane-1-carboxylate that acts as a linker was reacted with albumin in a phosphate buffer saline(pH 7.4) for 2 hours.

Non-reacted products were removed by using fast protein liquid chromatography and albumin bound to the linker was isolated. 7 mg of the albumin bound to the linker was reacted with 5 mg of peptide-Dox composite(Ac-Cys-Asp-Glu-Val-Glu-Ala-Pro-Lys-Dox) in a tris(2-carboxyethyl)phosphine-containing water for 12 hours, thereby forming albumin-peptide-anticancer drug nanoparticles that are an inactive anticancer prodrug.

### <Example 1> Cytotoxicity test

Each of Dox and albumin-peptide-Dox at different concentrations was added to HeLa cells and then a 3-(4,5-dimethylthiazol-2-yl)-2,5-diphenyltetrazolium bromide (MTT) assay was performed to evaluate cytotoxicity. As illustrated in FIG. 4, when Dox that is an anticancer drug was used, the level of cytotoxicity was increased in proportion to the concentration, but when the albumin-peptide-Dox that is the inactive anticancer prodrug was used, cytotoxicity did not occur.

### <Example 2> Anticancer effect

To evaluate an anticancer effect according to cell apoptosis, the anticancer prodrug prepared in Synthesis Example 1 was administered to an animal model in which cancer has been developed and then radioactive rays were irradiated to the animal model.

The cancer model was prepared by subcutaneously transplanting a squamous cell SCC7 cancer cell into a C3H/HeN mouse. First, 5 Gy of radioactive rays were irradiated to a cancer site to induce cell apoptosis. Then, two to three days later, the anticancer prodrug (200µg/mouse) prepared in Synthesis Example 1 was administered to the C3H/HeN mouse. After the radioactive rays were irradiated to the cancer site, the anticancer effect according to use of the anticancer prodrug prepared in Synthesis Example 1 was evaluated.

Like the cytotoxicity test, when radioactive rays were not irradiated, the anticancer prodrug prepared in Synthesis Example 1 did not show the anticancer effect. However, when 5 Gy of radioactive rays were irradiated to the cancer site, the anticancer effect occurred. Specifically, when radioactive rays that can induce cell apoptosis were irradiated to the cancer model to which the anticancer prodrug prepared in Synthesis Example 1 had been administered, the anticancer effect was distinctively increased compared to when only radioactive rays were irradiated.

Hereinafter, formulation examples of a pharmaceutical composition including an anticancer prodrug according to the present invention will be described. However, these formulation examples are used only to describe the present invention in detail and the present invention is not limited to these formulation examples.

### <Formulation Example 1> Preparation of injectable formulation

3 mg of the anticancer prodrug prepared in Synthesis Example 1 was mixed with 3.0 mg of sodium metabisulfite, 0.8 mg of methylparaben, 0.1 mg of propylparaben and an appropriate amount of an injectable sterile distilled water, and then the mixture was treated by using a conventional method until the final volume reached to 2 mℓ and a 2 mℓ-volume ample was filled therewith and sterilized, thereby manufacturing an injectable formulation.

### <Formulation Example 2> Preparation of tablet formulation

20 mg of the anticancer prodrug prepared in Synthesis Example 1, 100 mg of lactose, 100 mg of starch and an appropriate amount of magnesium stearate were mixed together and the mixture was compressed by using a conventional method of manufacturing a table formation, thereby manufacturing a tablet formulation.

### <Formulation Example 3> Preparation of capsule formulation

10 mg of the anticancer prodrug prepared in Synthesis Example 1, 50 mg of lactose, 50 mg of starch, 2 mg of talc and an appropriate amount of magnesium stearate were mixed together and a gelatin capsule was filled with the mixture by using a conventional method of manufacturing a capsule formation, thereby manufacturing a capsule formulation.

An anticancer prodrug according to the present invention is selectively accumulated at a target cancer site and is specifically reactive to a particular protease to release an active anticancer drug. Accordingly, an anticancer effect can be selectively made on cancer cells and thus, a therapeutic effect can be maximized and side effects of the anticancer treatment can be minimized.

## Claims

1. An anticancer prodrug sensitive to a target protease, comprising an anticancer drug that is an active component, peptide and a polymer, which are bound to each other,
wherein the peptide is specifically decomposed by the target protease excessively secreted by cancer cells, and the polymer is specifically accumulated at a target cancer site.

2. The anticancer prodrug of claim 1, further comprising a linker that binds the peptide bound to the anticancer drug to the polymer.

3. The anticancer prodrug of claim 1 or claim 2, wherein the peptide is decomposed by irradiation of radioactive rays and thus, the anticancer drug is released at the target cancer site.

4. The anticancer prodrug of claim 1 or claim 2, wherein the anticancer drug is any one selected from the group consisting of doxorubicin, paclitaxel, adriamycin, cisplatin, 5-fluorouracil, mitomycin, chlomomycin, bleomycin, peplomycin, daunorubicin, aclarrubicin, neocarzinostatin, epirubicin, idarubicin and pirarubicin.

5. The anticancer prodrug of claim 1 or claim 2, wherein the peptide is a peptide set forth in SEQ ID NO.1.

6. The anticancer prodrug of claim 1 or claim 2, wherein the polymer is any one selected from the group consisting of collagen, gelatin, dextran, poly-L-lysine, poly(N-2-(hydroxypropyl)methacrylamide, poly(divinyl ether-co-maleic anhydride), poly(styrene-co-maleic anhydride), and poly(ethylene glycol).

7. The anticancer prodrug of claim 1 or claim 2, wherein the target protease is any one selected from the group consisting of caspases, matrix metalloproteinases (MMP), thrombin, FXllla, caspase, urokinase plasminogen activator (uPA), HIV protease, DPP-IV and proteasome.
